# EUROPEAN PATENT APPLICATION

(11) **EP 2 241 245 A1**
(43) Date of publication of application: **20.10.2010**
(21) Application number: 09157879.9
(22) Date of filing: 14.04.2009
(51) Int. Cl.: A61B 5/00, A61B 5/0488

(54) **Using muscle tension detection to control position of body part**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

Apparatus for controlling movement of a body part has a muscle tension sensing device (1) arranged to sense muscle tension on a muscle of the body part, and a processor (2) for controlling an actuator to move the body part based on the sensed muscle tension, to a position which corresponds to a relaxed state of the muscle. This can help reduce movement of the body part and so enable any movement sensitive actions such as non invasive glucose measurements made on the body part to be more consistent, and more accurate.

## Description

### FIELD OF THE INVENTION

This invention relates to apparatus and methods for controlling a position of a body part based on sensing or detecting muscle tension, and to corresponding computer programs for carrying out such methods.

### BACKGROUND OF THE INVENTION

There are many factors which can interfere with measurements made on the skin of a body part to detect skin analyte(s) concentration non-invasively by means of optical, electrical and/or optoelectronic methods, e.g., non-invasive glucose monitoring. Currently a number of companies are developing instruments for non-invasive glucose measurements based on optical methods. A major problem comes from the varying optical properties of human skin tissue. Although these methods are proven to have sufficient sensitivity for in-vitro and/or ex-vivo glucose quantification, none of the currently existing companies have been successful in bringing a non-invasive device to the market. The main reason is that the accuracy and reliability of recently developed devices is not sufficient to get FDA approval.

Non-invasive measurement of skin analytes is the most desirable method for consumers. But the uncertainty and inaccuracy hampers the acceptance of non-invasive testing. There is a need in the non-invasive glucose-monitoring market to solve the inaccuracy and unreliability problems.

Also methods and instruments have been developed for minimally invasive measurement of physiological parameters in a human or animal body, such as, for example, glucose measurements based on optical methods. These methods make use of a sensor, e.g., microsensors, implanted beneath the skin which is in contact with subcutaneous fluids. The sensor may include gels, particles, and/or liquids which are biodegradable. Preferably, the sensor, or microsensor, that is implanted is small in size, and does not require a complicated or painful insertion below the skin. The present invention could increase the accuracy and reliability of the sensor or microsensor readout because it can detect muscle tension, inform the user to relax the muscle, indicate when the muscle is relaxed, and then the user can proceed with the analyte concentration measurement.

Typically, analyte measurements deal with a large number of chemical, physical, and physiological interferences that obscure the true analyte concentration. These chemical, physical and physiological interferences include, *inter alia,* the water and salt concentrations in the skin, the level of melanin in the skin, the temperature of the skin, and the movement of the skin from a tense muscle below the skin. Information about these interferences, their collective and individual effects on analyte measuring, and the variability of results they cause are not known.

It is known that measurements performed on human tissue are often corrupted by large variations of the measurement sample that do not relate to the desired target analyte. These disturbing and undesired variations are called interferences to the target analyte. Often times these large variations are due to the chemical and physiological interferences like the ones mentioned in the previous paragraph. One attempt to solve this issue is based on mathematical analyses of the measured data. Typical approaches apply chemometrical methods, (e.g., multivariate analysis), to extract the desired information from a measurement that is corrupted by these various interferences. Basically these chemometrical methods are about performing a post-processing of the measure data to filter out the interferences from the target signal. However, for such complex systems as human skin, a large number of interferences can have a large influence on the measured signal. Therefore, post-processing chemometric methods become very complex and are prone to large errors. The instant invention discloses a way to stabilize the human skin, (or the measurement volume), during the analyte measurement itself The apparatus and methods of the current invention can reduce or eliminate some of these interferences by informing the user when the muscle is relaxed and the measurement can be taken. Thus, the error correction for these interferences is moved from the post-processing phase into the signal acquisition phase. This will increase the reproducibility and accuracy of non-invasive analyte measurements taken from the skin.

### SUMMARY OF THE INVENTION

An object of the invention is to provide apparatus and methods for controlling a position of a body part, with or without actuators, based on electromyography, ("EMG"), that detects muscle tension, and to corresponding computer programs for carrying out such methods. According to a first aspect, the invention provides:
An Apparatus for controlling movement of a body part, the apparatus having a muscle tension sensing or detection device arranged to sense muscle tension on a muscle of the body part, and a processor for controlling an actuator to move the body part based on the sensed muscle tension, to a position which corresponds to a relaxed state of the muscle.

This can help reduce movement of the body part and so enable any "movement sensitive actions" made on the body part to be more consistent, and more accurate.

Embodiments of the invention can have any other features added; some such additional features are set out in dependent claims and described in more detail below.

Other aspects of the invention include corresponding methods, and computer programs for using muscle tension sensing signals to control moving a body part to a position which corresponds to a relaxed state of the muscle. Any of the additional features can be combined together and/or combined with any of the aspects of the invention. Other advantages will be apparent to those skilled in the art, especially over other prior art. Numerous variations and modifications can be made without departing from the claims of the present invention. Therefore, it should be clearly understood that the form of the present invention is illustrative only and is not intended to limit the scope of the present invention.

### BRIEF DESCRIPTION OF THE DRAWINGS

How the present invention may be put into effect will now be described by way of example with reference to the appended drawings, in which:
FIG. 1 shows a flow chart by which an apparatus according to a first embodiment works.
FIG. 2 shows a method according to an embodiment.
FIG. 3 shows a view of a forearm in a first lower position (A) on an actuator:
FIG. 4 shows a view of the same forearm in a second higher position (B) in which the forearm position is vertically changed by the actuator.
FIG. 5 shows the EMG signal from a muscle in the forearm at position A (solid-line) and position B (dashed-line).
FIG. 6 shows positions of a forearm moved horizontally between right and left positions.
FIG. 7 shows the EMG signal from a muscle in the forearm at position 1 (solid-line) and position 2 (dashed-line).
FIG. 8 shows an apparatus according to an embodiment.
FIG. 9 shows a method according to an embodiment.

### DETAILED DESCRIPTION OF EMBODIMENTS

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn to scale for illustrative purposes. Where the term "comprising" is used in the present description and claims, it does not exclude other elements or steps. Where an indefinite or definite article is used when referring to a singular noun e.g., "a" or "an", "the", this includes a plural of that noun unless something else is specifically stated.

The term "comprising", used in the claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention. References to a signal can encompass any kind of signal in any medium, and so can encompass an electrical or optical or wireless signal or other signal for example. References to analyzing can encompass processing a signal in any way to derive or enhance information about the material. References to a processor can encompass any means for processing signals or data in any form and so can encompass for example a personal computer, a microprocessor, analog circuitry, application specific integrated circuits, software for the same, and so on.

In the description provided herein, numerous specific details are set forth. However, it is understood that embodiments of the invention may be practiced without these specific details. In other instances, well-known methods, structures and techniques have not been shown in detail in order not to obscure an understanding of this description. Additionally, the term, and all forms of the term, "sense or sensing" is used synonymously herein with the term, and all forms of the term, "detect" or "detecting".

### Introduction to some issues addressed by some of the embodiments

By way of introduction to the embodiments, the problems of measuring an analyte through the skin, in particular, by minimally invasive and non invasive blood glucose monitoring, will be discussed briefly. The measurement of glucose through near-infrared spectroscopy is based on a change in the concentration of glucose being indicated by a change in the absorption of light according to the absorption and scattering properties of glucose and/or the effect of glucose changes upon the anatomy and physiology of the sampled site. However, in addition to the effect of glucose on the near-infrared light probing signal that is delivered to the skin, the probing signal is also reflected, diffusely reflected, transmitted, scattered, and absorbed in a complex manner related to the structure and composition of the tissue. When near-infrared light is delivered to the skin, a proportion of reflected light, or specular reflectance, is typically between 4-7% of the delivered light over the entire spectrum. Absorption by the various skin constituents accounts for the spectral extinction of the light within each layer. Scattering is the main process by which the beam may be returned to contribute to the diffuse reflectance of the skin. Scattering also has a strong influence on the light that is diffusely transmitted through a portion of the skin.

The scattering of light in tissues is in part due to discontinuities in the refractive indices on the microscopic level, such as the aqueous-lipid membrane interfaces between each tissue compartment or the collagen fibrils within the extracellular matrix. The spectral characteristics of diffuse remittance from tissue result from a complex interplay of the intrinsic absorption and scattering properties of the tissue, the distribution of the heterogeneous scattering components, and the geometry of the point(s) of irradiation relative to the point(s) of light detection.

The near-infrared absorption of light in tissue is primarily due to overtone and combination absorbances of the C-H, N-H, and O-H functional groups. As skin is primarily composed of water, protein, and fat; these functional groups dominate the near-IR absorption in tissue. As the main constituent, water dominates the near-infrared absorbance above 1100 nm and is observed through pronounced absorbance bands at 1450, 1900, and 2600 nm. Protein in its various forms, in particular, collagen is a strong absorber of light that irradiates the dermis. Near-infrared light that penetrates the subcutaneous tissue is absorbed primarily by fat. In the absence of scattering, the absorbance of near-infrared light due to a particular analyte, A, can be approximated by Beer's Law. An approximation of the overall absorbance at a particular wavelength is the sum of the individual absorbance of each particular analyte given by Beer's Law. The concentration of a particular analyte, such as glucose, can be determined through a multivariate analysis of the absorbance over a multiplicity of wavelengths because it is unique for each analyte. However, in tissue compartments expected to contain glucose, the concentration of glucose is at least three orders of magnitude less than that of water. Given the known extinction coefficients of water and glucose, the signal targeted for detection by reported approaches to near-infrared measurement of glucose, i.e., the absorbance due to glucose in the tissue, is expected to be, at most, three orders of magnitude less than other interfering tissue constituents. Therefore, the near-infrared measurement of glucose requires a high level of sensitivity over a broad wavelength range. Multivariate analysis is often utilized to enhance sensitivity, but often this sensitivity is still inadequate for accurate and reliable analyte measurement.

In addition, the diverse scattering characteristics of the skin, e.g., multiple layers and heterogeneity, cause the light returning from an irradiated sample to vary in a highly nonlinear manner with respect to tissue analytes, in particular, glucose. Simple linear models, such as Beer's Law have been reported to be invalid for the dermis.

Dynamic properties of the skin also add to the difficulties. Variations in the physiological state and fluid distribution of tissue profoundly affect the optical properties of the tissue layers and compartments over a relatively short period of time. For all these reasons therefore, the optical properties of the tissue sample are modified in a highly nonlinear and profound manner that introduces significant interference into noninvasive analyte measuring on, in, or under the skin.

The measurement of glucose through spectroscopy can also be made by a change in the absorption of light according to the absorption and scattering properties of minimally invasive microsensors, or to the change in light emitted or reflected from such microsensors located below the skin. Such methods using microsensors may include, for example,
- observing fluorescence (e.g., fluorescence resonance energy transfer) of a competitive binding assay encapsulated in microcapsules, for example based on competitive binding between the protein Concanavalin A and various saccharide molecules, specifically a glycodendrimer and glucose, the microcapsules can be polyelectrolyte microcapsules;
- detecting glucose using boronic acid-substituted viologens in fluorescent hydrogels in which a fluorescent anionic dye and a viologen are appended to boronic acid, which serve as glucose receptors, and are immobilized into a hydrogel, the fluorescence of the dye being modulated by the quenching efficiency of the viologen based receptor which is dependent upon the glucose concentration;
- other methods, e.g., monitoring oxygen or pH or other "smart tattoo" related methods.

The skin is a supple tissue. It tends to move while placing a measuring device on it. This skin movement makes accurate measurement of analytes such as glucose more difficult. It has now been appreciated that skin movement depends on muscle relaxation, and that this in turn depends on body posture, in the sense of position of the body part. But it is rather difficult for users, whether they are medical practitioners, or test subjects, to identify the proper position to relax the muscle in order to take an analyte measurement.

### Introduction to features of the embodiments

To reduce this movement, the body part position can be controlled as described below. Embodiments of this invention enable easier location of the proper position of the body part by using a muscle tension sensing array over the required measuring site. Embodiments can have any kind of muscle tension sensing device arranged to sense or detect muscle tension. Another feature is a processor of any kind for controlling an actuator to move the body part based on the detected muscle tension, to a position which corresponds to a relaxed state of the muscle.

The processor can comprise an electromyographic device. The processor can be arranged to move the position of the body part to minimize the detected electromyographic signals. The body part can be a forearm, and the actuator should be suitable for moving the joints of the forearm, or leg, as the case may be. The apparatus can have an indicator part coupled to the processor to indicate when the body part is in the relaxed state. The apparatus can also comprise a part of a measuring device for measuring a characteristic of the skin over the muscle, while the muscle is in the relaxed state.

A corresponding method involves detecting muscle tension of a muscle of the body part, and controlling an actuator to move the body part based on the detected muscle tension, to a position which corresponds to a relaxed state of the muscle. A subsequent step can be measuring a characteristic of the skin over the muscle, while the muscle is in the relaxed state. The measuring can involve moving a hand held minimally invasive or non invasive optical probe over the skin. The probe can be integrated with the detecting/sensing device, or with the part for indicating that the muscle is relaxed.

Some embodiments show a muscle contracting monitoring system containing sensors that detect a muscle contraction and via an appropriate algorithm can thereby adjust the subject's arm and/or leg position(s) by mechanical actuators according to the detected muscle contractions. When muscle tension is consistent during the minimally invasive or non-invasive analyte monitoring of the skin, these measurements can be more accurate and reliable. An example of the device has a means to manipulate extremity relaxation by mechanical actuators according to muscle contraction measurements via an appropriate algorithm. In some cases there are electrodes to measure the EMG signals. These signals are interpreted and sent to a processor. The actuators are controlled by the processor to adjust the extremity position until minimal EMG signals are observed. After minimal EMG signals are reached, the measurements and/or treatments are taken using a measuring device such as a testing module.

Embodiments using some of these features can address some of the uncertainty and inaccuracy which beleaguers the current methods of minimally invasive or non-invasive analyte measurement methods such as glucose monitoring. They can help meet the need in the minimally invasive or non-invasive glucose-monitoring market to solve the inaccuracy and unreliability problems that currently exist due to incorrect body posture, particularly the arms and legs.

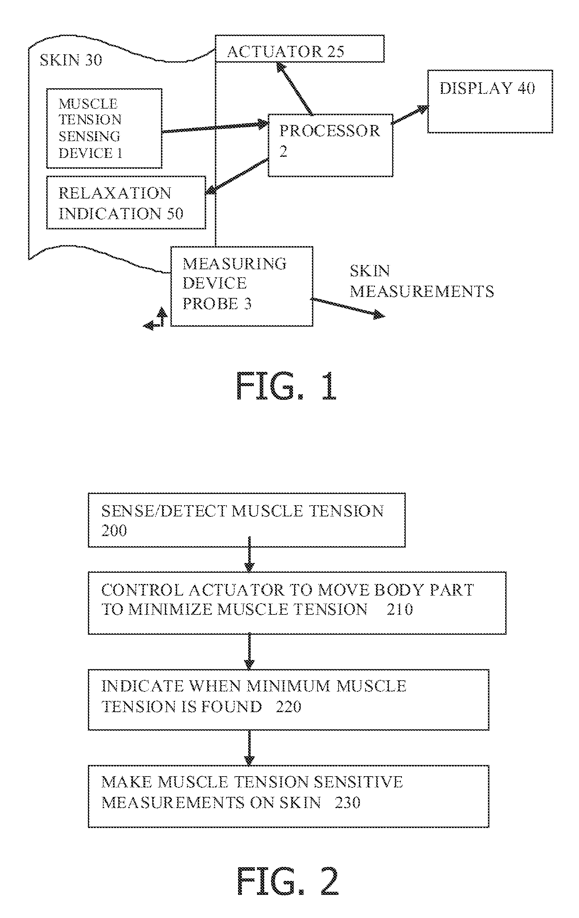

### FIGS. 1, 2, first embodiment of the invention

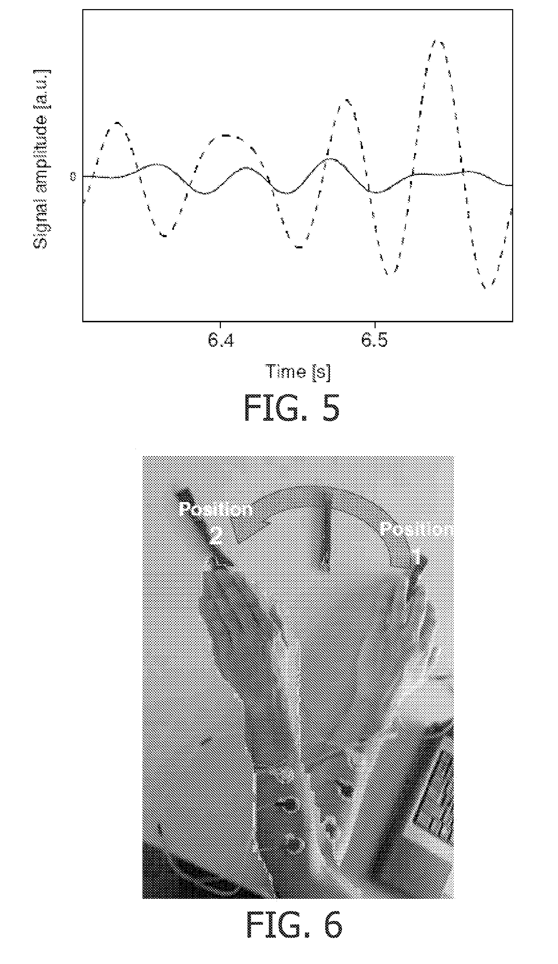

FIG. 1 shows a schematic view of an apparatus according to a first embodiment. On a surface of the skin 30 of a body part is a muscle tension sensing device 1, which may be in the form of electrodes on a pad stuck to the skin. Other types of devices can also be envisaged. Signals indicating the level of muscle tension are sent to a processor 2 for determining how to control an actuator 25. These signals that sense or detect muscle tension can be EMG signals as described in more detail below. Examples of these sensing signals are described below with reference to FIG. 5. The processor controls the actuator or actuators so as to minimize the sensing signals received because the muscle is becoming relaxed. It may determine and output an indication when the minimum signal is found. This indication can be on a display 40 or in some form on the skin itself, as shown by relaxation indication 50. This indication could be for example a projected image on the skin, or an array of LEDs on a skin patch. Also shown is a measuring device probe 3 able to take skin measurements. This can be minimally invasive or non invasive reflectance measurements to determine glucose measurements for example.

Once a body position corresponding to the minimum muscle tension is found, a reduced skin movement at the proper position is expected. Experimental proof provides evidence for the high correlation between muscle tension signals and accurate body position. Hence measurements should be taken once the most relaxed muscle position is detected.

FIG. 2 shows steps in a method using the apparatus of FIG. 1 or other embodiments. At a first step 200, muscle tension is detected. Next, the actuator moves the body part to minimize the detected muscle tension, at step 210. Optionally at step 220, an indication is provided when the minimum muscle tension is found. This enables a user to know when to start making skin analyte measurements. Alternatively, the measuring can be started automatically as soon as the minimum muscle tension is reached. Step 230 shows the making of measurements on the skin of the body part while the muscle is relaxed.

Muscle tension is a sign of uncomfortable patient posture and can lead to excessive, undesired movement during measurement and/or treatment. This and other embodiments can measure the muscle tension over part of the body extremities and can adjust the posture of the body extremities to minimize the muscle tension by mechanical actuators, via an appropriate algorithm. This way, the extremity muscles tend to relax and thus reduced body movement is expected. Experimental proof provides evidence for the high correlation between desired extremity position and muscle tension. It indicates that the extremity position can be optimized to non-invasively measure skin analytes by a muscle tension detection system, e.g., EMG measurement.

Human body posture is seen as a problem that prevents the reproducibility and accuracy of minimally invasive or non-invasive measurements due to undesired body movements. This and other embodiments can help to minimize the body movement, by manipulating extremities of the body to reach a position of relaxation to enable minimally invasive or non-invasive measurement of skin analytes.

### EMG systems

It is known to use EMG to use electrodes to stimulate muscles, focusing on pain relief. For more details reference is made to these patents showing details of conventional methods to implement such EMG sensing. For example US Pat. 4570640 "Sensory monitoring apparatus and method" shows monitoring the sensory system of a patient to enable determination of the level and depth of spinal and epidural nerve blocks, including those induced by anesthetics administered to a patient, which blocks affect the sympathetic and motor nervous systems. The anesthesia level is sequentially and repeatedly scanned at a plurality of spaced points to provide a continuous determination of the extent and depth of superficial and deep sensation and sympathetic and motor integrity. The electronic apparatus includes a stimulator that provides selective stimulation to each element of a multiple element transmitting unit the elements of which are non-invasively positioned contiguous to the skin of a patient. A physiological response detector is used to detect patient responses to stimulation sensed by the elements of a multiple element sensing unit the elements of which are also non-invasively positioned contiguous to the skin of the patient. A multiple display is provided to facilitate monitoring and an indication and/or termination of stimulation signal is provided whenever stimulation exceeds a reference level.

Reference is also made to US Pat. 6600954, entitled, "Method and apparatus for selective control of nerve fibers", which shows using a plurality of electrode devices spaced along the length of the nerve bundle which are sequentially actuated with delays corresponding to the velocity of propagation of the body-generated action potentials through the large-diameter fibers to produce a "green wave" effect which minimizes undesired anodal blocking of the large-diameter fibers while maximizing the collision blocking of the small-diameter fibers.

Reference is also made to EP 1637076 which shows detecting and processing EMG signals using a substrate having a bottom surface adapted for attachment to skin.

The EMG signal is composed of the action potentials (or electrical pulses) from groups of muscle fibers (grouped into functional units called motor units). Reference can be made to the book "Muscles Alive" (5 Th.Ed, 1985) for more details. The EMG signal is detected with electrodes placed on the surface of the skin or with needle or wire electrodes introduced into the muscle tissue. The term decomposition is commonly used to describe the process whereby individual motor unit action potentials (MUAPs) are identified and uniquely classified from a set of superimposed motor unit action potentials which constitute the EMG signal. A decomposed EMG signal provides all the information available in the EMG signal. The timing information provides a complete description of the inter-pulse interval, firing rate and synchronization characteristics. The morphology of the shapes of the MUAPs provides information concerning the anatomy and health of the muscle fibers.

US Pat. 5474083 relates to a lift monitoring and exercise training system which uses electromyographic sensor to monitor muscle force for lift training and exercise training. The exercise training embodiment has a bar graph displaying muscle intensity and two light emitting diodes alerting a user when to contract or relax a monitored muscle group. The microprocessor is programmed with an exercise routine and alerts a user when the routine is to begin, the duration of muscle contraction and relaxation, and the repetitions required. During exercise the user can monitor muscle intensity from the bar graph display and or auditory feedback element.

WO 2008036746 can be referred to for more details of how to implement a powered orthotic device which includes a brace for a limb. The device also includes an electromyographic (EMG) sensor and an actuator assembly in communication with the electromyographic sensor, the actuator assembly mounted to the brace, and occupying a volume of which a majority is disposed proximately to the outside region of the brace, and coupled to the first and second sections of the brace so as to apply a force for driving the first and second sections about the pivot, the is force based on signals from the electromyographic sensor.

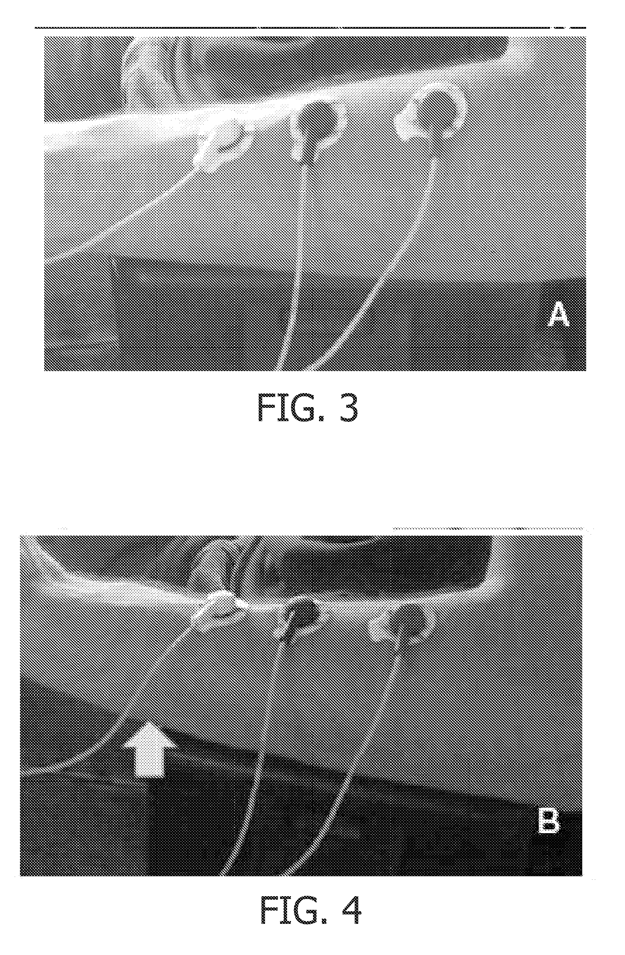

### FIGs. 3, 4, and 5 forearm positions with vertical movement

FIG. 3 shows a view of a forearm in a first lower position (A) on an actuator. FIG. 4 shows a view of the same forearm in a second higher position (B) in which the forearm position is vertically changed by the actuator. FIG. 5 shows the EMG signal from forearm at position A (solid-line) and position B (dashed-line).

FIGs. 3 and 4 show the forearm has a number of electrodes. An actuator is located below the arm to move the arm vertically. The EMG signals are recorded by a pair of "stick-on" electrodes placed over the forearm, and are sent to a processor using Moby8, a multi-channel biological signal processing device (TMSi, Twente). The sampling frequency is 2048 Hz, using high pass cut off filter of 6 Hz.

EMG signals are produced from muscle contraction/tension. Various EMG signals are acquired from a subject by changing the arm position horizontally and vertically, respectively.

Various EMG signals from a muscle within the forearm are recorded when the arm position is changed vertically (FIGs. 3 and 4). The EMG signals with varied amplitude at different positions are shown in FIG. 5.

FIG. 5 shows that the EMG signal at position B is much stronger than at position A. Coincidently the subject reported more fatigue at position B than at position A. The observations indicate a stronger muscle tension at position B. As a result, the body moves to compensate for the strong muscle tension. Therefore position A is desirable for the subject as the muscle is more relaxed. FIG. 5 provides an example of how large the difference can be in the EMG signals between a tense muscle and a relaxed muscle. The amplitude of the EMG signal can be quantified in Volts, e.g., µV, mV; in percentage of MVIC, in Mean amplitude of the rectified signal and/or RMS value of the signal.

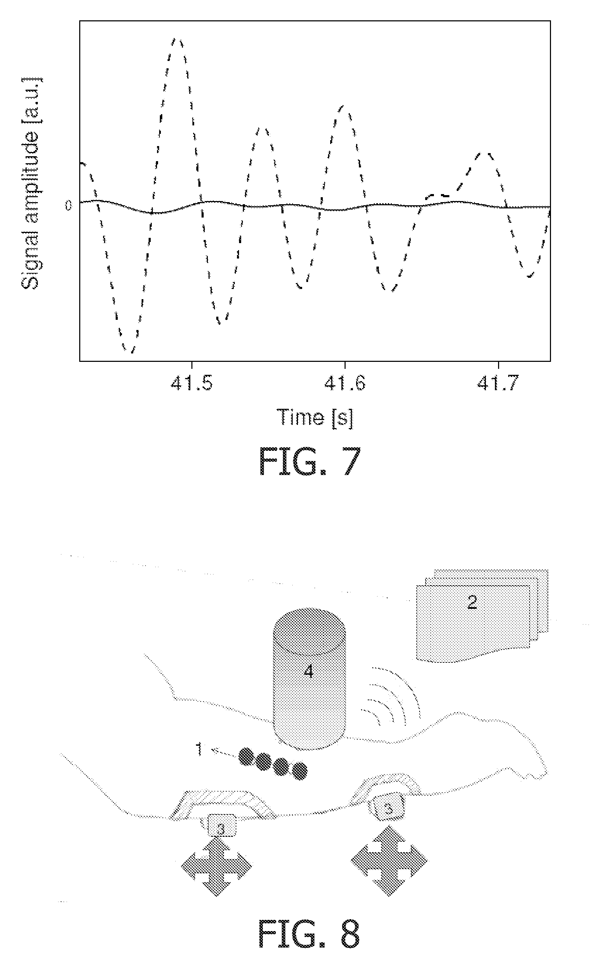

### FIGS. 6 and 7, horizontal movement

FIG. 6 shows positions of a forearm moved horizontally between right and left positions. FIG. 7 shows the EMG signal from forearm at position 1 (solid-line) and position 2 (dashed-line). Similar results to those shown in FIG. 5 are obtained when the forearm position is changed vertically.

### FIG. 8, apparatus embodiment

FIG. 8 shows an example of an apparatus according to an embodiment, incorporating a means to manipulate extremities to relax by mechanical actuators (3) according to detected muscle contraction signals, under the control of a processor (2) using an appropriate algorithm. The algorithm can be arranged to control the actuator to find a position for minimizing the muscle tension related signals. The electrodes (1) measure the EMG signals that indicate the level of muscle tension. These signals are interpreted and sent to the processor (2). The actuators (3) adjust the positions of the joints at each end of the forearm until minimal EMG signals are observed. After minimal EMG signals are obtained, the measurements and/or treatments are taken with a measurement device in the form of a testing module (4).

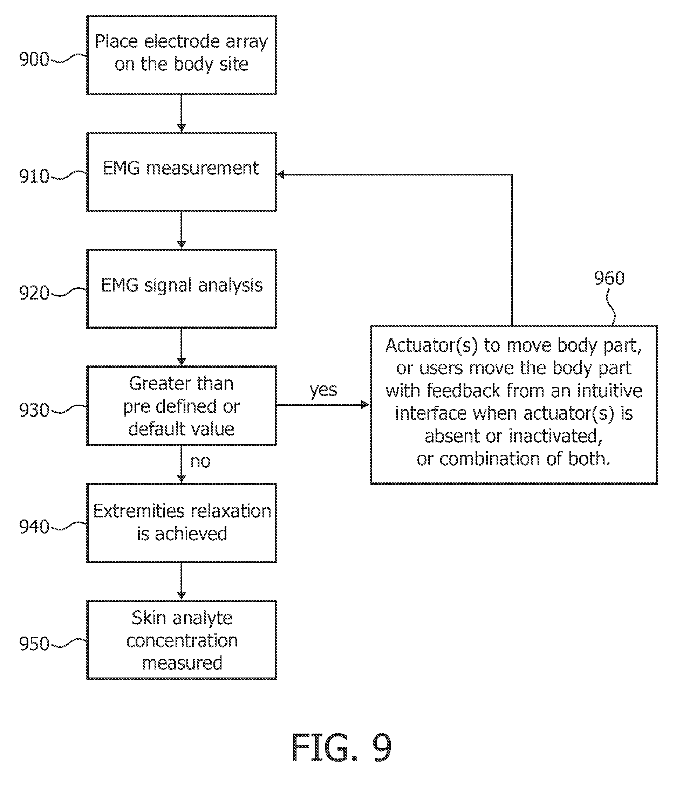

### FIG. 9, method embodiment

FIG. 9 shows steps in a method using the apparatus of FIG. 8 or other embodiments. At first step 900, the array of electrodes is placed along the body site horizontally relative to the length of the extremity. Next, at step 910, the EMG signal, which is an indication of the muscle tension, is measured. The EMG signal is analyzed at step 920. At step 930, the EMG signal is determined to be either greater than the predefined or default value or not greater than this value. If the EMG signal is determined to be greater than the predefined or default value, then at step 960 the actuators move the body part, or the user moves the body part based on feedback from an intuitive interface to where the EMG signal indicates a relaxed muscle, at step 940. If the EMG signal is determined to be lesser than, or equal to, the predetermined or default value for proper muscle tension, then step 940 is reached and the muscle is relaxed enough to proceed to step 950, i.e., measurement of the skin analyte concentration. The pre-determined or default value for optimum muscle tension as conveyed by the EMG signal can change from muscle to muscle, position to position, and person to person. It also depends on which kind of EMG sensor is used, e.g., monopolar, bipolar, etc. The predetermined or default value also depends on the size of the electrode, specific impedance of the senor, and depends on the amplifier used for the EMG sensor. The predetermined or default value should be in the range of micro-Volts to mini-Volts (peak-to-peak), e.g., 1 µV to 1 mV.

### Applications

The Apparatus, as proposed here, can be used by most techniques that measure analytes within any kind of skin. Embodiments of the invention can be used for minimally invasive or non-invasive glucose detection by means of NIR diffuse back-reflectance spectroscopy. Further applications include measurements of skin properties (e.g., skin cancer, skin aging, etc.) by means of light, or any measurements susceptible to interference by body part movement.

As has been described, many techniques have been investigated to detect skin analyte concentration minimally invasively or non-invasively by means of optical, electrical and/or optoelectronic methods, e.g., non-invasive glucose monitoring. The accuracy and reproducibility of these measurements are generally poor due to many inconsistent conditions like body posture, specifically increased muscle tension in the area to be tested. Incorrect body posture prevents the reproducibility and accuracy of minimally invasive or non-invasive measurements due to undesired body movements. The embodiments described can help to minimize body movement by influencing extremity relaxation during minimally invasive or non-invasive measurement by using a muscle tension detection system. Optimizing body posture to provide extremity relaxation can lead to minimizing body movement, decrease muscle tension and improve skin analyte concentration measurements.
Other variations can be envisaged within the scope of the claims.

## Claims

1. An apparatus for controlling movement of a body part, the apparatus having a muscle tension sensing device (1) arranged to sense muscle tension on a muscle of the body part, and a processor (2) for controlling an actuator to move the body part based on the sensed muscle tension, to a position which corresponds to a relaxed state of the muscle.

2. The apparatus of claim 1, wherein the processor comprises an electromyographic device.

3. The apparatus of claim 2, wherein the processor communicates with at least one actuator to move the position of the body part to minimize the sensed electromyographic signals.

4. The apparatus of any preceding claim, wherein the body part is a forearm and the actuator is suitable for moving joints of the forearm.

5. The apparatus of any preceding claim comprising an indicator part coupled to the processor to indicate when the muscle is in the relaxed state.

6. The apparatus of any preceding claim comprising a part of a measuring device for measuring a characteristic of the skin over the muscle, while the muscle is in the relaxed state.

7. A method of controlling movement of a body part, the method having the steps of sensing muscle tension on a muscle of the body part, and controlling an actuator to move the body part based on the sensed muscle tension, to a position which corresponds to a relaxed state of the muscle.

8. The method of claim 7 wherein the sensing step involves the use of electromyographic sensing.

9. The method of claim 8 wherein the control of movement of the body part involves moving the position of the body part to minimize the sensed electromyographic signals.

10. The method of any of claims 7 to 9 comprising the step of providing an indication to a user when the muscle is in the relaxed state.

11. The method of any of claims 7 to 10 comprising the step of measuring a characteristic of the skin over the muscle, while the muscle is in the relaxed state.

12. The method of claim 11 wherein the measuring involves moving a hand held non invasive optical probe over the skin.

13. The method of claim 11 or 12, wherein the measuring involves measuring reflectance and determining the level of glucose in the skin, based on the reflectance.

14. A computer program on a computer readable medium which when executed by a computer, is arranged to control an actuator to move a body part based on sensed muscle tension, to a position which corresponds to a relaxed state of the muscle.

15. A method of controlling movement of a body part, the method having the steps of placing electrodes horizontally along an extremity length, taking an EMG signal measurement, analyzing the EMG signal as to whether the EMG signal is greater than or less than the predetermined or default value for relaxed muscle tissue, wherein the user moves the body part according to an intuitive interface to achieve muscle relaxation so analyte concentration measurements can be taken more reliably.
